# EUROPEAN PATENT SPECIFICATION

(11) **EP 0 431 519 B1**
(45) Date of publication and mention of the grant of the patent: **15.06.1994**
(21) Application number: 90123100.1
(22) Date of filing: 03.12.1990
(51) Int. Cl.: A61L 15/44, A61K 47/10

(54) **System for transdermal albuterol administration**
System zur transdermalen Albuterol Applikation
Système pour l'administration transdermale d'albutérol

(30) Priority: 04.12.1989 US 425766
(43) Date of publication of application: 12.06.1991
(73) Proprietor: G.D. Searle & Co., Chicago Illinois 60680 (US)
(72) Inventor: Farhadieh, Bahram, Libertyville, Illinois 60048 (US); Gokhale, Rajeeve Dattatrey, Vernon Hills, Illinois 60061 (US)
(74) Representative: Beil, Hans Chr., Dr.

(56) References cited:
- EP-A- 0 255 485
- EP-A- 0 344 840
- WO-A-87/01291
- US-A- 4 699 777
- US-A- 4 814 173
- PATENT ABSTRACTS OF JAPAN vol. 12, no. 209 (C-504)(3056) 15 June 1988,& JP-A-63 10716 (TEIJIN LTD) 18 January 1988,

## Description

The present invention comprises a transdermal patch for the administration of drugs percutaneously. In particular, the invention is useful for the administration of the drug albuterol, a β₂ adrenergic agonist, which is useful, among other things, in the treatment of asthma by virtue of its action of inducing bronchodilation.

The practicality of administering a given drug percutaneously on a continuous basis depends upon the concentration of drug in the blood that is required to provide the desired pharmacologic effect, the degree to which the skin is permeable to the drug, and the amount of skin surface area that is available for drug administration.

The skin surface area which is available for drug administration, while theoretically being unlimited, is, for practical reasons, typically confined to a range of from about five square centimeters to about 100 square centimeters. With the available skin surface area fixed within this range, the matter then narrows as to whether sufficient drug will pass through that much skin surface area to provide the desired therapy. If it will, then it may not be difficult to effectively administer the drug percutaneously. If, however, the inherent permeability of the skin to the drug is so high or so low that too much or too little drug will pass through that area of skin, then the rate of administration of the drug to the skin must be controlled, or the permeability of the skin to the drug must be increased, as the case may be, to make percutaneous administration practical.

The present invention involves a drug delivery system in which the percutaneous administration of the active drug component is enhanced by the presence of a diffusion enhancer.

Systemically active drugs are conventionally administered either orally or by injection, with the primary objective of either mode being to achieve a given desired blood level of drug in circulation over a period of time.

These prior conventional methods of administering drugs to patients, however, possess certain shortcomings resulting in the failure to this goal.

The oral route of drug administration, for example, is inadequate for several reasons, even if the drug is administered to the patient at periodic intervals according to a well-defined schedule.

The rate of absorption of drug through the gastrointestinal tract is affected by both the contents in the tract and the passage of time as the drug travels through the small intestine. Therefore, such variables as whether the drug is administered before or after eating, and the type and quantity of food eaten, for example, high or low fat content, or whether the drug is administered before or after a bowel movement, affect the rate of absorption of the drug which takes place in the small intestine.

Additionally, the time of passage of drug through the small intestine is affected by the rate of peristaltic contraction, adding further uncertainty.

Also important is the rate of circulation of blood to the small intestine, and the fact that many drugs administered by this route are rendered inactive by gastric acid and digestive enzymes of the gastrointestinal tract or liver, where the drug can be metabolized to an inactive product.

These factors make it difficult to achieve a desired time course of concentration of drug in the blood.

The most widely-used dosage form of albuterol, an orally-administered, instant-release (IR) tablet, is administered to a patient every 6 hours. The controlled-release (CR) albuterol tablet is administered to a patient every 12 hours.

A significant disadvantage associated with the oral administration of albuterol is that orally-administered albuterol undergoes extensive first pass metabolism, probably in the gastrointestinal tract, with the result that the bioavailability of the drug formulation is reduced from a potential bioavailability of 100 percent to as low as 10 percent.

The most inevitable result of the oral administration of drugs through the gastrointestinal tract is that the level of drug in circulation surges to a peak level at the time the drug is administered, followed by a decline in drug concentration in the blood and body compartments. Thus, a plot of a drug in circulation versus time after the administration of several tablets of the drug per day will have the appearance of a series of peaks which may surpass the toxic threshold of the drug, and valleys which may fall below the critical point needed to achieve the desired pharmacologic or therapeutic effect.

The administration of drugs by injection likewise entails certain disadvantages. For example, very strict asepsis must be maintained in order to avoid infection of the blood, the vascular system and the heart. Drug administration by poor intravenous injection technique may result in perivascular injection, when that was not intended. The typical result of injection of a drug into the blood is a sudden rise in the blood concentration of the drug followed by an uncontrollable decline in drug concentration. Additionally, administration of drugs by injection is inconvenient and painful.

Other dosage forms for systemic administration of drugs, such as rectal suppositories and sublingual lozenges, also produce non-uniform levels of the therapeutic agent in circulation. These dosage forms require great patient cooperation and have low patient acceptability, resulting in decreased patient compliance with a prescribed drug regimen, which is the most common failure of drug therapy.

To avoid the problems discussed above, a new branch of drug delivery has developed in which systemically-active drugs are administered through the skin or mucosa of a patient. Uncertainties of administration through the gastrointestinal tract, and the inconvenience of administration by injection, are decreased or eliminated by this system of drug administration. Because a high concentration of drug never enters the body, problems with pulse entry are overcome.

Despite these advantages of administering systemically-active drugs through the skin, many problems exist with prior art devices designed for this purpose. Many such devices do not provide a continuous administration of drug to the patient, or a continuous delivery rate.

Researchers working in the art have conducted studies of the effects of vehicles containing substances which materially affect skin penetration. A range of agents has been recorded as having accelerant action, in particular propylene glycol, surface active agents, dimethylsulfoxide, and dimethylacetamide. These substances, however, have certain drawbacks, including skin irritation potential. Their use to date has been limited.

B. Idson, Cosmetics and Toiletries 95, 59 (1980), has concluded that the factors affecting drug penetration into the skin and, consequently, in most cases effectiveness, are complex. The vehicle that provides ideal conditions for one drug may prove unsatisfactory for another.

The present invention seeks to overcome prior problems with the continuous administration of a drug to a patient, and with the delivery rate of the drug in general, and has been found to work particularly well with adrenergic agonists, and especially well with albuterol, a selective β₂ adrenergic agonist.

Another object of this invention is to provide a device for the administration of albuterol to a patient, in a reliable and easily-applied device for continuously administering the drug to the patient in controlled quantities through the patient's intact skin or mucosa.

Another object of this invention is to provide for such a drug delivery device which will cause little, if any, dermal or mucosal irritation to the patient.

Another object of this invention is to provide a drug delivery device which will be especially useful and acceptable in pediatric patients and geriatric patients.

A further object of this invention is to provide for a unitary, non-lamellar, single-layered drug delivery device.

Yet another object of this invention is to provide a drug administration device which will provide a continuous dozing of the drug to the patient over a 24-hour period.

The transdermal drug administration patches of the present invention generally provide a continuous administration of drug to the patient. In addition, these patches generally cause little or no dermal or mucosal irritation to the patient. Both of these qualities are significant advantages of the patches of the present invention.

Prior to the invention of the transdermal albuterol patches described herein, researchers working in the field were unable to successfully deliver albuterol to a patient by means of a transdermal patch.

The transdermal albuterol patches of the present invention feature, in addition to the benefits already described above, a 100% skin bioavailability of drug to a patient, a good margin of safety in pediatric and geriatric patients and ease of administration.

Albuterol administered transdermally through a patch of the invention is useful for actual asthma therapy, rather than merely for prophylaxis. It is also useful in both pediatric age groups and geriatric populations, both of which require simple-to-administer regimens that do not rely on the responsibility or memory of the patient to comply with daily dosage administrations of the drug, as is often needed with conventional tablets or capsules of albuterol. Transdermal albuterol therapy would also be useful after the treatment of an acute asthma attack to prevent the exacerbation of such an attack. Clinically, it would also be useful either as a substitute for intravenous therapy or as an improvement over oral therapy.

In addition to being convenient, transdermal albuterol therapy has a significant margin of safety. Significantly, an on-going therapy, such as with sustained-release oral formulations, could be interrupted if the average plasma level of the drug were too high. Once the patient was stabilized at a lower plasma level of drug, the transdermal albuterol patch would be beneficial to maintain consistent plasma levels of albuterol at a more desirable lower level.

Additionally, albuterol can be used transdermally as a tocolytic (obstetric) agent. Preterm labor occurs in approximately 10% of pregnancies. Commonly, beta-mimetic agents are employed for preterm labor. Albuterol is currently used for preterm labor, with the plasma albuterol levels needed for uterine relaxation being 8 to 33 nanograms of drug per milliliter. Such levels are within the range of albuterol delivered by the transdermal albuterol patches of the present invention.

The albuterol patches, of the present invention also have the potential advantage of safety over the intravenous route of drug administration, and the further advantage of a more uniform dosing of the drug to the patient in comparison with the oral route of drug administration, during the sensitive and critical period during which labor occurs. Such a use, of an albuterol patch of the invention may be adjunctive with bed rest and intravenous and oral agents, or may be primary therapy as a substitution for intravenous beta-mimetic agents.

Additionally, a transdermal albuterol patch of the invention may find usage as an emergency therapy for the treatment of urticaria (hives).

The usefulness of albuterol as a bronchodilator is not limited to the treatment of asthma. Albuterol can also be used as a bronchodilator in the treatment of bronchitis, chronic obstructive pulmonary disease and other obstructive pulmonary diseases.

### DESCRIPTION OF THE DRAWINGS

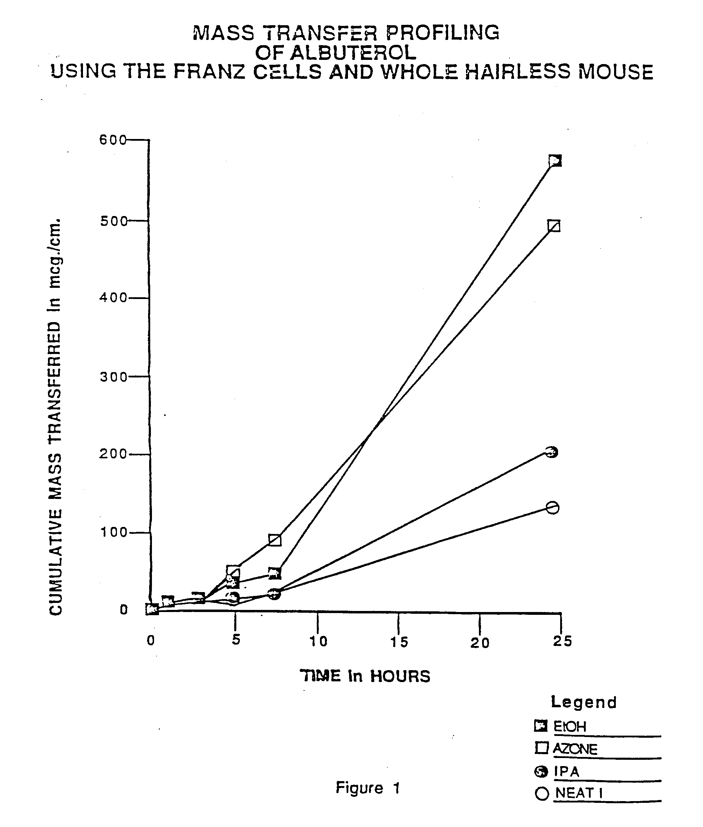

Figure 1 is a plot of the in vitro transfer of albuterol transdermally through the skin of a hairless mouse contained in a Franz Diffusion Cell Assembly (Example 1 ) versus time, showing how various adjuvant agents affect the flux of albuterol through the mouse skin. It can be seen that ethanol was the most effective agent when compared with prior art agents, such as Azone and isopropyl alcohol (IPA).

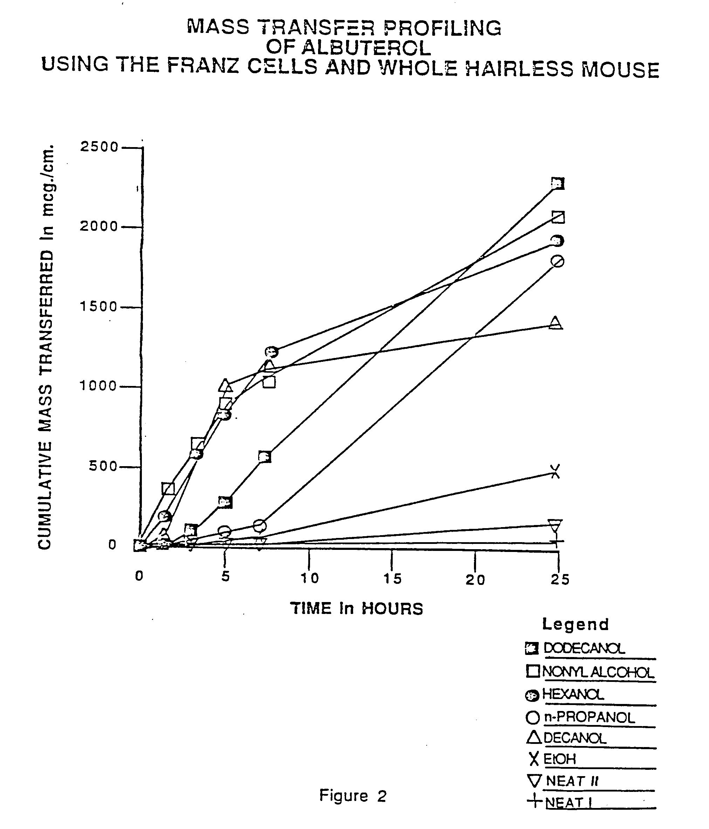

Figure 2 is the same type of plot as Figure 1, except that the agents tested are normal hydrocarbon alcohols. It can be seen that, in general, agent effect on albuterol flux increases as the chain length of the alcohol increases.

### SUMMARY OF THE INVENTION

The present invention comprises a transdermal patch for the administration of albuterol percutaneously.

The most preferred embodiment of this invention is a non-laminated monolayer patch for the transdermal administration of a drug to a patient comprising an elastomeric matrix material of predetermined thickness and area; an active drug ingredient dispersed throughout the matrix; and a diffusion enhancer being a normal hydrocarbon alcohol of 1 to 20 carbon atoms dispersed throughout the matrix.

Additionally, a suitable plasticizer and/or a solubilizing agent for the active ingredient can conveniently be incorporated into the patch.

### Elastomeric Matrix Materials

Suitable elastomeric matrix materials for use in the patches of the invention comprise the following polymers:
Polyethylene,
Polypropylene,
Polyethylene terephthalate,
Polyvinylidene fluoride,
Polymethyl methacrylate,
Polyurethane-polyamide copolymers,
Poly(2-hydroxyethyl methacrylate)
(HEMA-hydrogel),
Polyalkyl acrylate esters (bioadhesive polymers),
Polyisobutylene (bioadhesive polymer),
Polydimethylsilicone with resin (bioadhesive polymer), and Silicone elastomers.

The patches of the present invention preferably utilize a silicone elastomer as the matrix. Silicone elastomers have alternating silicon and oxygen atoms for a backbone. Double bonds are generally absent in such a backbone and, therefore, the numerous forms of stereoisomers ordinarily found in unsaturated hydrocarbon rubbers do not have counterparts in the silicone rubbers. An especially useful silicone elastomer for use in the patches of the invention is Silastomer X7-3058, available from Dow Corning, Inc., Midland, Michigan.

In the present invention, the active drug ingredient is albuterol, most preferably as the free base.

### Diffusion Enhancers

The diffusion enhancers for use in the patches of the invention are normal hydrocarbon alcohols of 1 to 20 carbon atoms with the most preferable diffusion enhancer being n-dodecanol, dispersed throughout the elastomeric matrix.

The patches as described above are useful for preparing a medicament for administering albuterol for treating skin or mucosal areas for treating bronchial constriction, urticoria or delaying premature uterine contractions.

A preferred embodiment according to the present invention is a double layer patch comprising an albuterol layer comprising albuterol and a silicone elastomer and a diffusion layer comprising a silicone elastomer, n-dodecanol and a catalyst as described in present example 3.

### DETAILED DESCRIPTION OF THE INVENTION

### Permeation of Drug through the Matrix Material

The present invention comprises a transdermal patch which is suitable, by virtue of the rate-controlling materials employed therein, for the predetermined controlled administration of drug to the skin or mucosa of a mammal over a period of time. The patch of the invention is applied to the patient's skin or mucosa and should be in firm contact therewith so as to form a tight seal. Flow of drug from the patch to the patient's skin or mucosa is metered through the matrix material of the patch in accordance with the laws of diffusion, as hereinafter discussed, at a predetermined rate. In operation, drug molecules are continuously removed from the patch, migrating through the patch to the skin or mucosa of the patient, where the drug is absorbed and enters the patient's circulation through the capillary network.

The rate of passage or permeation of drug through the elastomeric matrix material of the patch is determined by the diffusive flux of the drug molecules through the material, as is the case where the material is of a solid nature in which the drug molecules can dissolve in, and flow through, to a direction of lower chemical potential.

The release rate of the drug can be controlled in accordance with "Fick's First Law," depending on the design of the particular drug transfer mechanism, which may vary according to certain variables, such as the diffusivity and solubility of the drug being employed in the diffusive medium, and the thickness of the matrix material of the patch.

The mechanism of action of the diffusion enhancers described herein may be to increase the diffusivity of active ingredient through the patch matrix material. This mechanism of action shall be understood to attach to the term "diffusion enhancer" as used herein.

### Elastomeric Matrix Materials

Preferred elastomeric matrix materials for use in the patches of the invention are the organopolysiloxane rubbers, commonly known as silicone rubbers. Suitable silicone rubbers are the conventional heat vulcanizable (curable) silicone rubbers and the room temperature vulcanizable silicone rubbers. Room temperature vulcanizable silicone rubbers will require the use of a curing agent or catalyst. The most especially preferred silicone rubber for use in the patches of the invention is Silastomer X7-3058, available from Dow Corning, Inc. Other room temperature vulcanizable silicone rubbers suitable for use in the patches of the invention are also commercially available.

Exemplary patents disclosing the preparation of silicone rubbers are U.S. Patent Nos. 2,541,137; 2,723,966; 2,863,846; 2,890,188; 2,927,907; 3,002,951 and 3,035,016.

Elastomer can be present in the patches of the invention in an amount ranging from 25 to 95 per cent, weight to weight. More preferably, it can be present in an amount ranging from 65 to 90 per cent, weight to weight.

### Catalysts

Catalysts which may be employed to cure the elastomeric matrix material component of the patches of the invention include stannous 2-ethyl hexoate.

Catalyst can be present in the patches in a range of from 0.0625% to 0.5%.

### Diffusion Enhancers

A diffusion enhancer is employed in the patches of the present invention, being the normal hydrocarbon alcohols of one to twenty carbon atoms. As the chain length of the alcohol increases, the effectiveness of the diffusion enhancer generally increases up to a point. The most preferred diffusion enhancer for use in the patches of the invention is n-dodecanol. n-dodecanol can be present in an amount ranging from 3 to 30 per cent, weight to weight. More preferably, it can be present in an amount ranging from 10 % to 15 per cent, weight to weight.

### Plasticizers

Plasticizers are useful for increasing the plasticity of polymers. In a preferred embodiment of the patches of the present invention, a suitable plasticizer is employed. Preferred plasticizers include diols, triols, and other polyols. The cost preferred plasticizer is glycerol.

### Solubilizing Agents

In a preferred embodiment of the patches of the present invention, a solubilizing agent for the active ingredient is employed. Preferred solubilizing agents include the normal hydrocarbon alcohols, with n-hexanol being the most preferred solubilizing agent. n-hexanol can also act as a useful plasticizer.

### Active Agents

The most preferred patch of the present invention comprises, out of 100%, Dow Silastomer X7-3058:Dow X7-3059 crosslinking agent (97.86:2.14), 71,9%, w/w; albuterol, 16 % w/w; n-dodecanol, 10 % w/w; glycerol, 1.75%, w/w; and hexanol, 0.35%, w/w; with a suitable organotin catalyst, 0,1 % w/w (generally stannous 2-ethyl hexoate).

All materials used in the patches of the present invention are dispersed uniformly throughout the matrix material employed therein. This dispersion results from the use of an elastomeric matrix material.

The amount of active agent incorporated within the elastomeric matrix material of the patches of the invention to obtain the desired therapeutic effect will vary depending upon the desired dosage of the active agent, the length of time the patch is to remain on the skin or the body mucosa of the patient and the area and thickness of the patch. Patient serum concentrations of the active agent can thus be adjusted by varying the concentration of the active agent in the patch, the length of time the patch is to remain on the skin or body mucosa of the patient or the patch size.

Because the patches of this invention are designed to control drug administration for an extended period of time, ideally 24 hours or more, absent toxicity concerns, there is no critical upper limit on the amount of active agent incorporated into the patches. The lower limit of the amount of active agent incorporated into the patches is determined by the fact that sufficient amounts of the agent must remain in the patches to maintain the desired dosage of the agent for the particular patient being treated.

In order to achieve a therapeutic effect of albuterol in a human adult suffering from a condition which is treatable with albuterol the patient serum concentration of albuterol should be in the range of between 2 and 33 nanograms of albuterol per milliliter of serum, and most preferably between 4 and 8 nanograms per milliliter. From 4 to 8 nanograms of albuterol per milliliter is desirable for treating bronchoconstriction, and from about 8 to about 33 nanograms of albuterol per millillter is desirable for using albuterol as a tocolytic agent.

The effective rate of release of the active agent from the patches of the invention to the skin or mucosa of a patient can be in the range of from 0.2 to 2.0 milligrams of active agent per square centimeter of skin or mucosa per day (mg.cm⁻².day⁻¹). A more preferred range would be from 0.3 to 0.85 milligrams of active agent per square centimeter of skin or mucosa per day. The exact amount will depend on the desired dosage of the active agent, as well as the condition being treated.

Those skilled in the art can readily determine the rate of permeation of active drug ingredient through a particular matrix material, and through selected combinations of matrix materials, to be employed in a patch of the invention. Standard techniques employed for making such determinations are described in the Encyclopedia of Polymer Science and Technology, Volumes 5 and 9, Pages 65 to 85 and 795 to 807 (1968), and the references cited therein.

Albuterol can be present in the patches of the invention in an amount ranging from 2 to 30 per cent, weight to weight. More preferably, it can be present in an amount ranging from 8 to 24 per cent, weight to weight, and most preferably, in an amount ranging from 12 to 20 per cent, weight to weight.

### Backing Members

Various occlusive and non-occlusive, flexible and non-flexible backing members can be used in the patches of the present invention, if desired.

Suitable backing members for use in the patches of the invention include cellophane, cellulose acetate, ethylcellulose, plasticized vinylacetate-vinylchloride copolymers, polyethylene terephthalate, nylon, polyethylene, polypropylene, polyvinylidenechloride, paper, cloth, foam and aluminum foil.

### Protective Release Films and Foils

To prevent the passage of drug away from the exposed surface of a patch of the invention prior to its use, the surface of the patch generally can be covered with a protective release film or foil, such as waxed paper.

To enhance the stability of the active compound(s) employed in a patch of the invention, the patch is usually packaged between hermetically-sealed polyethylene terephthalate films or aluminum foils under an inert atmosphere, such as gaseous nitrogen.

### Application of Patches

The patches of the invention are applied to the skin of patients. A patch should be in firm contact with the patient's skin, preferably forming a tight seal therewith. Drug within the patch will, then migrate through the patch to the patient's skin by diffusion. When drug is in contact with the patient's skin, drug molecules which are continuously being removed from the outer surface of the patch migrate through, and are absorbed by, the skin, entering the patient's circulation through the capillary network. The patch can be applied to any area of the patient's skin, including the oral mucosa, for example, by application of the patch to the patient's palate or buccal mucosa. In addition, the patches of the invention can be used to administer drugs to other mucosa of the body, for example, by application to the vaginal mucosa, the rectal mucosa, etc.

### Example 1

Franz cell experiments. An eight week old male hairless mouse was sacrificed by spinal dislocation and a rectangular piece of abdominal skin was carefully lifted and separated from the adhering fatty tissue and visceral material. The skin tissue was mounted and clamped between the donor and the receptor compartments of a Franz cell with the epithelium facing the donor compartment. The temperature of the receptor was maintained by the external water bath set at 37° C, and the receptor solution was stirred with a magnetic stirrer. The receptor compartment was then charged with the normal saline solution, bathing the dermis of the skin tissue. Thus, the dermis was washed to remove the adhering cell debris. After two hours, the receptor solution was withdrawn and replaced with the fresh saline solution. Following this, a 1.38 square centimeter portion of the pad was cut and glued (355 Medical adhesive, Dow Corning, Midland, MI) on the adhesive foam (Fasson, Painville, OH). The delivery system containing the pad and an adhesive foam was then applied on the epidermal side of the skin. At the predetermined time intervals, 300 microliter samples of the receptor solution were removed from the sampling port, and replaced with the equivalent volume of normal saline solution. The samples were filtered, and quantitated by an HPLC method. Concentrations were converted into amounts, accounting for the dilution factor which was the volume of the receptor solution (7 ml). The amounts were then normalized for the area (1.38 cm²) to calculate skin permeation rates (mg.cm⁻².day⁻¹) (Table I).

Pad residue analysis experiments: Pad residue analysis was conducted simultaneously with the pharmacokinetic experiments in monkeys.

Dissolution Experiments: A 8 square centimeter pad was cut and mounted on a holder of the Hansen's dissolution test apparatus, exposing 4 square centimeter area. The test apparatus was assembled and the test conducted using water as dissolution medium at 37°C. The dissolution medium was stirred at 50 rpm and five milliliter samples removed from the sampling port at the periodic intervals. These were then analyzed by an High Performance Liquid Chromatographic Method (HPLC). The chromatographic peaks were quantitated using a peak height method to determine concentrations. These were then converted into amounts, accounting for the dilution factor which was volume of the dissolution medium (300 ml). The amounts were normalized for the area (4 cm²) to calculate the dissolution rates (mg. cm⁻².day⁻¹) (Table II).

After overnight fasting, monkeys were restrained in chairs and chest areas clipped to remove hair. Skin surface was then wiped clean with the isopropyl alcohol solution. A four square centimeter portion of the pad was then cut and glued on the center of the adhesive backing. The delivery system containing these two components was then applied on the chest area, pressing gently for proper adhesion. After 24 hours, the delivery system was carefully removed from monkeys, and the pad separated from the adhesive backing. Initial albuterol content was estimated from the weight of the pad, the content uniformity, and percent loading. The residual content in the pad was determined by extracting the pad with acetone. The extracts were analyzed by an HPLC method to determine residual albuterol in the pad. From the initial and final albuterol content in the pad, its loss and hence the in vitro release rates (mg./cm² .day) were calculated. These are listed in Table III.

Serum concentration time profiles after intravenous administration: Four female rhesus monkeys, #388, #391, #423, and #430, were used in a cross over design for IV and pad bioavailability experiments. From each monkey, 7 ml of blood sample was removed on the previous day of the experiment, centrifuged to separate serum, and stored at -20°C till the analysis. This serum sample was used to make blanks and standards. Following this, monkeys were fasted overnight, restrained and settled in chairs before the intravenous injection. Albuterol was dissolved in normal saline (0.9% sodium chloride) solution in such a way that the dose was 50 mcg/kg for 1ml/kg injection. Albuterol solution were then injected into the saphenous vein. Five milliliter blood samples were removed at 0.00, 0.08, 0.17, 0.33, 0.75, 1.00, 1.5, 2.00, 3.00, 4.00, and 5.00 hours following 50 mcg/kg injection. The serum was separated from blood via centrifugation and stored at -20°C till analysis.

Serum concentration time profiles after transdermal pad administration. After overnight fasting, on the day of the experiment, monkeys were restrained in chairs and the chest areas were clipped to remove hair, avoiding any injury to the skin tissue. Skin surface was wiped with isopropyl alcohol swab and a 4 square centimeter portion of the pad was then applied to the chest area for 24 hours and was removed as described previously. Following the pad application, blood samples were removed at 0.00, 0.5, 1.00, 1.50, 3.00, 5.00, 7.00, 12.00, 24.00, 31.00, and 48.00 hours, centrifuged to separate serum and stored at -20°C till the analysis.

Analysis of serum samples. Serum albuterol and internal standard bamethan sulphate were extracted into chloroform to remove polar interfering substances and reextracted in to the aqueous phase to eliminate nonpolar materials. The aqueous extracts were analyzed by an HPLC-Fluorescence method. Pharmacokinetic parameters were estimated by conventional pharmacokinetic methods well known to those of ordinary skill in the art.
Results: Following IV injection of 50 mcg/kg dose of albuterol, the initial half lives obtained were 6.0 minutes. Similarly, the terminal half lifes was 135.6 minutes.

The volume of the central compartment was 362.46 ml/kg after 50 mcg/kg doses. Serum drug concentrations observed after 50 mcg/kg I.V. dosing are listed in table IV.

The serum concentration time data and pharmacokinetic parameters of albuterol obtained after transdermal pad application are shown in tables V and VI. The time course of transdermal albuterol showed a steady incline up to 12 hours (tₛₛ) following single layer pad application. Thereafter, the steady-state concentrations were maintained till the pad was removed at 24 hours. In monkeys 423 and 430, however, after application of a single layer pad, albuterol concentrations were higher at 24 hours than at 12 hours. Since blood samples were not withdrawn between these time intervals, it was difficult to establish the time to achieve steady-state (tₛₛ). In all the monkeys, however, drug concentrations declined rapidly after the pad was removed, with no measurable concentration remaining at 48 hours. Comparison of in vitro release rates obtained from the pad residue analysis and in vivo absorption rates K₀ calculated from pharmacokinetic parameters is given in table VII. Comparison between in vitro - in vivo parameters of albuterol pads is given in table VIII. Hypothetical serum concentrations were calculated from K₀ and clearance (C1) in monkeys and then extrapolated to a 70 kg human (table IX).

Dose versus area under the curve relationships following intravenous and transdermal application of albuterol is given in table X.

| HAIRLESS MOUSE SKIN PERMEATION RATE CONSTANTS. |
|---|
| (mg.cm⁻².day⁻¹) |
| Single Layer Pad |
| 0.50, 0.53, 0.48 |
| Mean: 0.50 |
| S.D.: 0.02 |

| PAD DISSOLUTION RATE CONSTANTS |
|---|
| (mg.cm⁻².day⁻¹) |
| Single Layer Pad |
| 2.25, 2.19, 2.39 |
| 2.28, 2.31, 2.50 |
| Mean: 2.34 |
| S.D.: 0.14 |

**TABLE III**

| Residual pad analysis after application to monkey skin. | |
|---|---|
| Parameter | Single Layer Pad |
| Initial Amount (mg.cm⁻²) | 6.26(2.30)¹) |
| Residual Amount (mg.cm⁻²) | 5.50(1.98) |
| In vitro release rate (mg.cm⁻².day⁻¹) | 0.76(0.28) |

| | |
|---|---|
| 1. Number in parentheses is standard deviation of the mean. | |

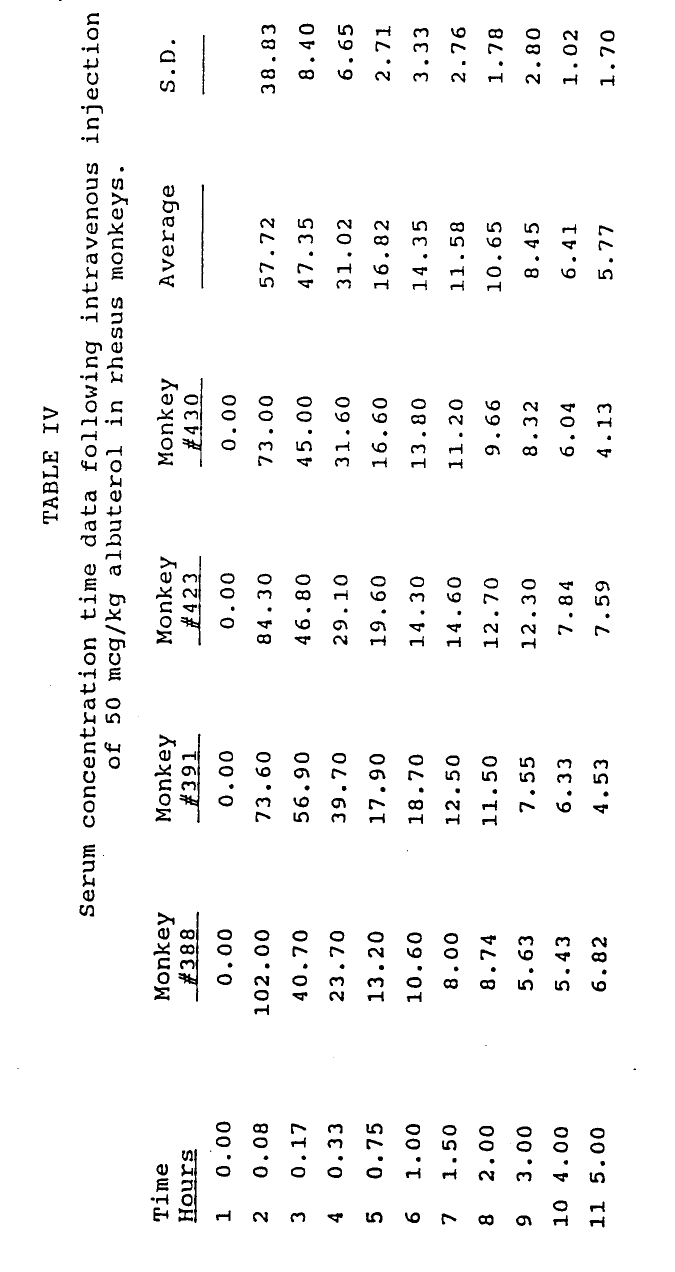

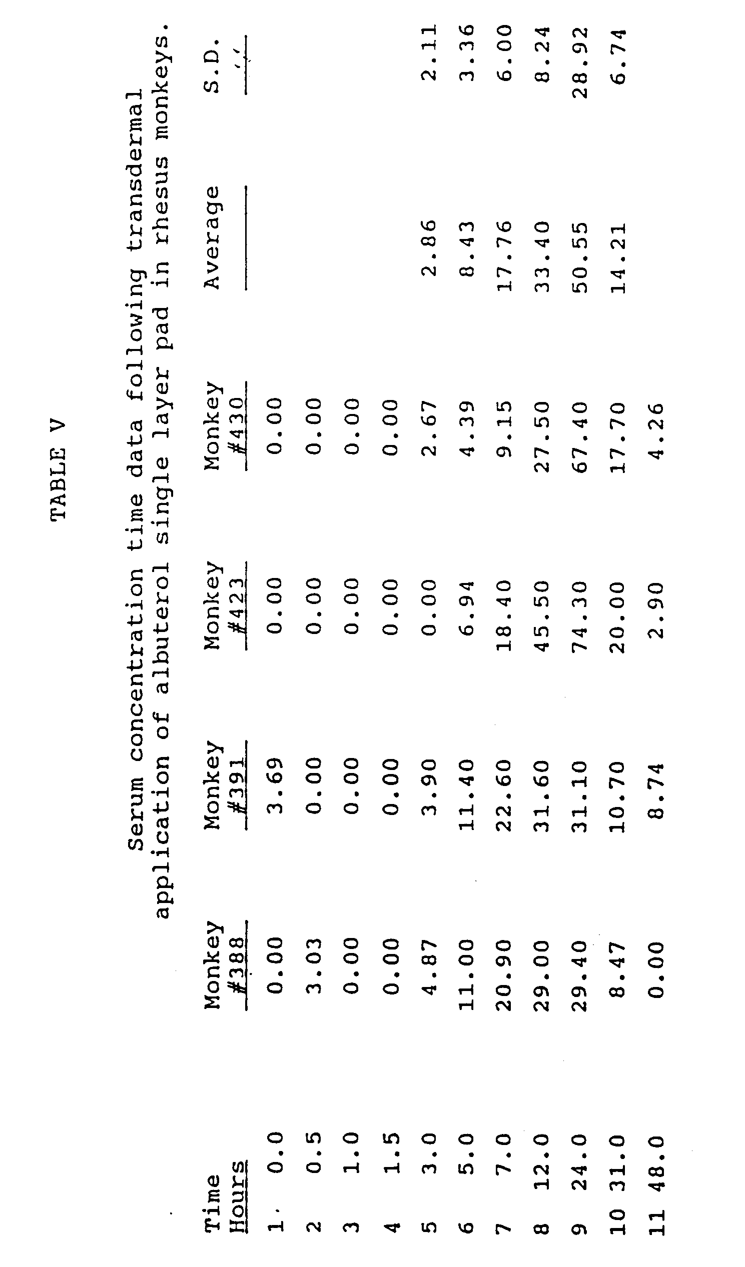

**TABLE VI**

| Mean and standard deviation of pharmacokinetic parameters obtained after transdermal application of albuterol single layer pads in rhesus monkeys (n = 4) | | |
|---|---|---|
| Parameter | Mean | SD |
| Weight | 5.39 | 0.025 |
| AUC (ng.ml⁻¹.hrs) | 1070.6 | 394.10 |
| Css (12-24 hrs) (ng.ml⁻¹) | 42.0 | 14.45 |
| Kₒ (mg.day⁻¹.cm⁻²) | 0.81 | 0.20 |
| AUC = Area Under Curve Cₛₛ = Serum Concentration at Steady State Cl = Clearance Rate Kₒ = In Vivo Absorbtion Rate Constant | | |

**TABLE VII**

| Comparison of in vitro-in vivo parameters obtained following pad residue analysis and in vivo absorption rates of albuterol single layer pads in monkeys. | | |
|---|---|---|
| Subject | In vitro release rate (mg.cm⁻².day⁻¹) | Kₒ (mg.cm⁻².day⁻¹) |
| Monkey #388 | 0.52 | 0.68 |
| Monkey #391 | 0.53 | 0.60 |

**TABLE VIII**

| Comparison of in vitro parameters of albuterol pads. | | |
|---|---|---|
| Parameter (mg.cm⁻².day⁻¹) | Single Layer | |
| | Mean | S.D. |
| Dissolution rate constant. | 2.34 | 0.14 |
| Hairless mouse skin permeation rate constant. | 0.50 | 0.02 |
| Monkey skin permeation rates constant | 0.76 | 0.28 |

**TABLE IX**

| Hypothetical serum concentrations after application of albuterol pads in a 70 KG human. | |
|---|---|
| Concentration (ng.ml⁻¹) Pad Size (cm²) | Serum Single Layer |
| 4.0 | 2-4 |
| 8.0 | 4-8 |
| 16.0 | 8-16 |

**TABLE X**

| Dose versus area under the curve comparison following intravenous and transdermal application of albuterol in rhesus monkeys. | | | | |
|---|---|---|---|---|
| Parameter | Monkey Identification | | | |
| | #388 | #391 | #423 | #430 |
| Intravenous dose (mg.Kg⁻¹) | 0.05 | 0.05 | 0.05 | 0.05 |
| Transdermal dose (mg.Kg⁻¹) | 0.38 | 0.39 | 0.77 | 0.72 |
| AUC Area Under the Curve after intravenous administration (ng.ml⁻¹. hr) | 70.69 | 85.72 | 106.39 | 73.96 |
| AUC Area under the Curve after transdermal administration (ng.ml⁻¹. hr) | 711.51 | 777.2 | 1534.52 | 1259.29 |

### EXAMPLE 2

100 g of albuterol pad formulation was prepared with 71.90 g Dow X7-3058 Silastomer , 16 g albuterol, 10 g n-dodecanol, 1.75 g glycerol, 0.35 g hexanol and X7-3075 catalyst, available from Dow Corning.

In a clean mortar, catalyst was mixed with silastomer in a geometric dilution.

In a clean mortar, a fine paste of albuterol, dodecanol, hexanol and glycerol was made. One portion of the silastomer-catalyst blend was mixed thoroughly with the albuterol paste. Following this, the remaining two portions of the silastomer-catalyst blend were mixed one at a time with the albuterol paste.

The resulting mass was passed through a triple roller mill to obtain a homogeneous mixture. The mixture was then placed between two sheets of mylar plastic film and passed through twin aluminum rollers of a film casting apparatus well known to those of ordinary skill in the art. The thickness of the spread can be adjusted by manipulating the gap between the two rollers. The resulting spread was then cured in an oven at 100°C for ten minutes.

The cured film was cut into four square centimeter pieces and each piece was glued onto a 4 square cm patch of aluminum foil. The surface of the foil not in contact with the pad was then adhered onto a patch of adhesive-lined foam.

### EXAMPLE 3

A double layer formulation can be made, having two separate albuterol and dodecanol layers.

100 g of albuterol layer mixture prepared was with 40 g of Dow X7-3058 Silastomer , 20 g albuterol, 5.0 g glycerol 1.0 g hexanol and catalyst.

100 g of dodecanol layer was prepared with 90 g of Dow X7-3058 Silastomer , 10 g dodecanol and catalyst.

For each layer, catalyst was mixed with Silastomer in a clean mortar.

In a clean mortar a fine paste of albuterol, hexanol and glycerol was made. One portion of the first silastomer-catalyst mixture was mixed thoroughly with the albuterol paste. Following this, the remaining two portions of the first silastomer-catalyst mixture are mixed one at a time with the albuterol paste.
The dodecanol layer was prepared by mixing the dodecanol with the other silastomer-catalyst mixture.

The resulting masses of the albuterol and dodecanol layers were separately processed as follows. Each mass was passed through a triple roller mill to obtain a homogeneous mixture. Each mixture was then placed between two sheets of mylar plastic film and passed through two aluminum rollers of a film casting apparatus. Each spread was then cured in an oven at 100°C for 10 minutes.

The cured films of albuterol and dodecanol layers were cut into pieces, each measuring four square centimeters. The dodecanol layer was placed on top of the albuterol layer. The side of the dodecanol layer not in contact with the albuterol layer was glued to a four square centimeter patch of aluminum foil. The surface of the foil not in contact with the dodecanol layer was then adhered to a patch of adhesive-lined foam.

## Claims

1. A monolayer patch for the transdermal administration of albuterol comprising:
a. an elastomeric matrix, said elastomeric matrix being present in an amount ranging from 25 to 95 per cent, weight to weight;
b. albuterol, said albuterol being present in an amount ranging from 2 to 30 per cent, weight to weight; and
c. a diffusion enhancer, said diffusion enhancer being a normal hydrocarbon alcohol having from 1 to 20 carbon atoms, and said diffusion enhancer being present in an amount ranging from 3 to 30 per cent, weight to weight.

2. The patch according to Claim 1 wherein said elastomeric matrix is present in an amount ranging from 65 to 90 per cent, weight to weight.

3. The patch according to Claim 1 wherein said elastomeric matrix is a silicone elastomer.

4. The patch according to claim 3 wherein said silicone elastomer is Silastomer X7-3058.

5. The patch according to each of Claims 1 to 4 wherein said diffusion enhancer is n-dodecanol.

6. The patch according to Claim 5 wherein said n-dodecanol is present in an amount ranging from 10 to 15 per cent, weight to weight.

7. The patch according to Claim 6 wherein said albuterol is present in an amount ranging from 8 to 24 per cent, weight to weight.

8. The patch according to Claim 7 wherein said albuterol is present in an amount ranging from 12 to 20 per cent, weight to weight.

9. The patch according to Claim 1, additionally comprising a plasticizer.

10. The patch according to Claim 9 wherein said plasticizer is a polyol.

11. The patch according to Claim 10 wherein said polyol is glycerol.

12. The patch according to Claim 11, additionally comprising a solubilizer.

13. The patch according to Claim 12, wherein said solubilizer is a normal hydrocarbon alcohol.

14. The patch according to Claim 13 therein said hydrocarbon alcohol is n-hexanol.

15. The patch according to any of Claims 1 to 12, additionally comprising a curing catalyst for said elastomeric matrix.

16. The patch according to Claim 15 wherein said Silastomer X7-3058 is present in an amount of 71,90 %, weight to weight, said albuterol is present in an amount of 16 %, weight to weight, said n-dodecanol is present in an amount of 10 %, weight to weight, said glycerol is present in an amount of 1,75 %, weight to weight, said n-hexanol is present in an amount of 0.35%, weight to weight, and said organotin catalyst is present in an amount of 0,1 %, weight to weight.

17. The patch according to Claim 15, additionally comprising a backing member.

18. The patch according to Claim 17, additionally comprising a protective release film and/or a low adhesion back side coating.

19. Use of a patch according to Claim 1, 3, 4, 5, or 6 for preparing a medicament for administering albuterol to a mammal in need thereof to the skin or mucosal areas of said mammal.

20. Use of a patch according to Claim 1, 3, 4, 5, or 6 for preparing a medicament for treating bronchial constriction and/or urticaria in a mammal in need thereof by applying it to the skin or mucosal areas of such mammal.

21. Use of a patch according to Claim 1, 3, 4, 5, or 6 for preparing a medicament for delaying premature uterine contractions in a pregnant mammal in need thereof by applying it to the skin or mucosal areas of said mammal.

22. A double layer patch for the transdermal administration of albuterol comprising an albuterol layer and a diffusion enhancer layer, said albuterol layer comprising:
a. an elastomeric matrix, said elastomeric matrix being X7-3058 Silastomer in an amount of 40 %, weight to weight, and
b. albuterol, said albuterol being present in an amount of 20 %, weight to weight, and
c. 5 % glycerol, weight to weight, 1 % hexanol, weight to weight and a catalyst,
and said diffusion enhancer layer comprising:
a. an elastomeric matrix, said elastomeric matrix being X7-3058 Silastomer in an amount of 90 %, weight to weight, and
b. a diffusion enhancer, said diffusion enhancer being dodecanol in an amount of 10% and a catalyst.

## Patentansprüche

1. Ein einlagiges Pflaster für die transdermale Verabreichung von Albuterol, enthaltend:
a. eine elastomere Matrix, wobei diese Matrix in einer Menge von 25 bis 95 %, Gewicht zu Gewicht, vorliegt;
b. Albuterol, wobei dieses Albuterol in einer Menge von 2 bis 30 %, Gewicht zu Gewicht, vorliegt und
c. einen Diffusionssteigerer, wobei dieser Diffusionssteigerer ein normaler Kohlenwasserstoffalkohol mit 1 bis 20 C-Atomen ist, und dieser Diffusionssteigerer in einer Menge von 3 bis 30 %, Gewicht zu Gewicht, voliegt.

2. Das Pflaster nach Anspruch 1, worin diese elastomere Matrix in einer Menge von 65 bis 90 %, Gewicht zu Gewicht, vorliegt.

3. Das Pflaster nach Anspruch 1, worin diese elastomere Matrix ein Silikonelastomer ist.

4. Das Pflaster nach Anspruch 3, worin dieses Silikonelastomer Silastomer X7-3058 ist.

5. Das Pflaster nach einem der Ansprüche 1 bis 4, worin dieser Diffusionssteigerer n-Dodecanol ist.

6. Das Pflaster nach Anspruch 5, worin dieses n-Dodecanol in einer Menge von 10 bis 15 %, Gewicht zu Gewicht, vorliegt.

7. Das Pflaster nach Anspruch 6, worin dieses Albuterol in einer Menge von 8 bis 24 %, Gewicht zu Gewicht, vorliegt.

8. Das Pflaster nach Anspruch 7, worin dieses Albuterol in einer Menge von 12 bis 20 %, Gewicht zu Gewicht, vorliegt.

9. Das Pflaster nach Anspruch 1, das zusätzlich einen Weichmacher enthält.

10. Das Pflaster nach Anspruch 9, worin dieser Weichmacher ein Polyol ist.

11. Das Pflaster nach Anspruch 10, worin dieses Polyol Glycerin ist.

12. Das Pflaster nach Anspruch 11, das zusätzlich einen Löslichmacher enthält.

13. Das Pflaster nach Anspruch 12, worin dieser Löslichmacher ein normaler Kohlenwasserstoffalkohol ist.

14. Das Pflaster nach Anspruch 13, worin dieser Kohlenwasserstoffalkohol n-Hexanol ist.

15. Das Pflaster nach einem der Ansprüche 1 bis 12, das zusätzlich einen Härtekatalysator für diese elastomere Matrix enthält.

16. Das Pflaster nach Anspruch 15, worin dieses Silastomer X7-3058 in einer Menge von 71,90 %, Gewicht zu Gewicht, dieses Albuterol in einer Menge von 16 %, Gewicht zu Gewicht, dieses n-Dodecanol in einer Menge von 10 %, Gewicht zu Gewicht, dieses Glycerin in einer Menge von 1,75 %, Gewicht zu Gewicht, dieses n-Hexanol in einer Menge von 0,35 %, Gewicht zu Gewicht, und dieser Organozinnkatalysator in einer Menge von 0,1 %, Gewicht zu Gewicht vorliegt.

17. Das Pflaster nach Anspruch 15, das zusätzlich eine rückwärtige Verstärkung enthält.

18. Das Pflaster nach Anspruch 17, das zusätzlich einen Freigabeschutzfilm und/oder eine rückseitige Beschichtung mit geringer Adhäsion enthält.

19. Verwendung eines Pflasters gemäß Ansprüchen 1, 3, 4, 5 oder 6 zur Herstellung eines Medikamentes zur Verabreichung von Albuterol an einen Säuger, der dies benötigt, auf die Haut- oder Schleimhautbereiche dieses Säugers.

20. Verwendung eines Pflasters gemäß den Ansprüchen 1, 3, 4, 5 oder 6 zur Herstellung eines Medikamentes zur Behandlung von Bronchialverengung und/oder Urtikaria eines Säugers, der dies benötigt, durch Aufbringung desselben auf die Haut-oder Schleimhautbereiche eines solchen Säugers.

21. Verwendung eines Pflasters gemäß den Ansprüchen 1, 3, 4, 5 oder 6 zur Herstellung eines Medikamentes zur Verzögerung verfrühter Uteruskontraktionen in einem schwangeren Säuger, der dies benötigt, durch Aufbringung desselben auf die Haut-oder Schleimhautbereiche dieses Säugers.

22. Ein doppellagiges Pflaster für die transdermale Verabreichung von Albuterol, enthaltend eine Albuterol-Lage und eine diffusionssteigernde Lage, wobei diese Albuterol-Lage enthält:
a. eine elastomere Matrix, wobei diese Matrix X7-3058 Silastomer ist, in einer Menge von 40 %, Gewicht zu Gewicht, und
b. Albuterol, wobei dieses Albuterol in einer Menge von 20 %, Gewicht zu Gewicht, vorliegt, und
c. 5 % Glycerin, Gewicht zu Gewicht, 1 % Hexanol, Gewicht zu Gewicht, und einen Katalysator,
und wobei diese diffusionssteigernde Lage enthält:
a. eine elastomere Matrix, wobei diese Matrix X7-3058 Silastomer ist, in einer Menge von 90 %, Gewicht zu Gewicht, und
b. einen Diffusionssteigerer, wobei dieser Diffusionssteigerer Dodecanol in einer Menge von 10 % und ein Katalysator ist.

## Revendications

1. Timbre monocouche destiné à l'administration transdermique d'albutérol, comprenant :
a. une matrice élastomère, ladite matrice élastomère étant présente en une quantité comprise entre 25 et 95% en poids ;
b. de l'albutérol, ledit albutérol étant présent en une quantité comprise entre 2 et 30% en poids et
c. un amplificateur de diffusion, ledit amplificateur de diffusion étant un alcool d'hydrocarbure normal ayant de 1 à 20 atomes de carbone et ledit amplificateur de diffusion étant présent en une quantité comprise entre 3 et 30% en poids.

2. Timbre selon la revendication 1, dans lequel ladite matrice élastomère est présente en une quantité comprise entre 65 et 90% en poids.

3. Timbre selon la revendication 1, dans lequel ladite matrice élastomère est un élastomère de silicone.

4. Timbre selon la revendication 3, dans lequel ledit élastomère de silicone est du Silastomer X7-3058.

5. Timbre selon chacune des revendications 1 à 4, dans lequel ledit amplificateur de diffusion est du n-dodécanol.

6. Timbre selon la revendication 5, dans lequel ledit n-dodécanol est présent en une quantité comprise entre 10 et 15% en poids.

7. Timbre selon la revendication 6, dans lequel ledit albutérol est présent en une quantité comprise entre 8 et 24% en poids.

8. Timbre selon la revendication 7, dans lequel ledit albutérol est présent en une quantité comprise entre 12 et 20% en poids.

9. Timbre selon la revendication 1, comprenant en outre un plastifiant.

10. Timbre selon la revendication 9, dans lequel ledit plastifiant est un polyol.

11. Timbre selon la revendication 10, dans lequel ledit polyol est un glycérol.

12. Timbre selon la revendication 11, comprenant en outre un agent de solubilité.

13. Timbre selon la revendication 12, dans lequel ledit agent de solubilité est un alcool d'hydrocarbure normal.

14. Timbre selon la revendication 13, dans lequel ledit alcool d'hydrocarbure est du n-hexanol.

15. Timbre selon l'une quelconque des revendications 1 à 12, comprenant en outre un catalyseur de durcissement destiné à ladite matrice élastomère.

16. Timbre selon la revendication 15, dans lequel ledit Silastomer X7-3058 est présent en une quantité de 71,90% en poids, ledit albutérol est présent en une quantité de 16% en poids, ledit n-dodécanol est présent en une quantité de 10% en poids, ledit glycérol est présent en une quantité de 1,75% en poids, ledit n-hexanol est présent en une quantité de 0,35% en poids et ledit catalyseur d'organo-étain est présent en une quantité de 0,1% en poids.

17. Timbre selon la revendication 15, comprenant en outre un renfort dorsal.

18. Timbre selon la revendication 17, comprenant en outre un film protecteur anti-adhésif et/ou un revêtement dorsal à faible adhérence.

19. Utilisation d'un timbre selon les revendications 1, 3, 4, 5 ou 6 pour préparer un médicament destiné à l'administration d'albutérol à un mammifère en ayant besoin, sur la peau ou des zones muqueuses dudit mammifère.

20. Utilisation d'un timbre selon la revendication 1, 3, 4, 5 ou 6, pour préparer un médicament destiné au traitement de la bronchoconstriction et/ou de l'urticaire chez un mammifère en ayant besoin, par son application sur la peau ou des zones muqueuses d'un tel mammifère.

21. Utilisation d'un timbre selon la revendication 1, 3, 4, 5 ou 6, pour préparer un médicament destiné à retarder les contractions utérines prématurées d'un mammifère gravide en ayant besoin, par son application sur la peau ou des zones muqueuses dudit mammifère.

22. Timbre double couche destiné à l'administration transdermique d'albutérol, comprenant une couche d'albutérol et une couche d'amplificateur de diffusion, ladite couche d'albutérol comprenant :
a. une matrice élastomère, ladite matrice élastomère étant du Silastomer X7-3058 en une quantité de 40% en poids et
b. de l'albutérol, ledit albutérol étant présent en une quantité de 20% en poids et
c. 5% en poids de glycérol, 1% en poids d'hexanol et un catalyseur,
et ladite couche d'amplificateur de diffusion comprenant :
a. une matrice élastomère, ladite matrice élastomère étant du Silastomer X7-3058 en une quantité de 90% en poids et
b. un amplificateur de diffusion, ledit amplificateur de diffusion étant du dodécanol en une quantité de 10% et un catalyseur.
